# EUROPEAN PATENT SPECIFICATION

(11) **EP 4 138 979 B1**
(45) Date of publication and mention of the grant of the patent: **18.02.2026**
(21) Application number: 21724128.0
(22) Date of filing: 20.04.2021
(51) Int. Cl.: A61M 27/00

(54) **DRAINAGE SHUNT DEVICES FOR PERITONEAL CAVITIES**
DRAINAGE-SHUNT-VORRICHTUNGEN FÜR PERITONEALHÖHLEN
DISPOSITIFS DE DÉRIVATION DE DRAINAGE POUR CAVITÉS PÉRITONÉALES

(30) Priority: 23.04.2020 US 202063014204 P
(43) Date of publication of application: 01.03.2023
(73) Proprietor: CAREFUSION 2200, INC, San Diego, CA 92130 (US)
(72) Inventor: PULITI, Gianluca, San Diego, California 92130 (US); SHEN, Hong, San Diego, California 92130 (US); HURTIS, Kelly, San Diego, California 92130 (US); FLANAGAN, Briana, San Diego, California 92130 (US)
(74) Representative: Hoffmann Eitle
(86) International application number: PCT/US2021/028097
(87) International publication number: WO 2021/216507

(56) References cited:
- US-A- 5 037 385
- US-A1- 2019 232 029

## Description

### CROSS REFERENCE TO RELATED APPLICATIONS

This application claims priority to U.S. Provisional Patent Application No. 63/014,204, filed on April 23, 2020.

### TECHNICAL FIELD

The present specification generally relates to drainage shunt devices for draining ascites fluid from peritoneal cavities and, more specifically, to peritoneal shunt devices to drain non-malignant ascites fluids for draining non-malignant ascites fluids.

### BACKGROUND

Shunt devices are used to drain fluid from an area within the body. Specifically, peritoneal shunt devices are used to drain ascites fluid from the peritoneal cavity of a subject. Ascites is the accumulation of fluid within the abdomen. In order to drain the fluid from the peritoneal cavity, shunt devices may rely on electronic pumps which provide consistent pressure to pump the fluid from the peritoneal cavity to the central venous system or the bladder of the subject. However, since the pump would always be activated in order to drain the fluid, the battery life of the pump diminishes, and needs recharging. If the pump were to stop functioning, no fluid would be drained from the peritoneal cavity, causing complications to the subject. Even further, blockages may form within the shunt devices as fluid passes through them. In order to clear the blockages within the shunt, varying pressures must be produced within the shunt.

By way of example, US 2019/0232029 A1 relates to a fluid management system using an electromechanical pump for the treatment of intracorporeal fluid accumulation, such as ascites.

Accordingly, there is a need for peritoneal shunt devices that drain fluid from the peritoneal cavity and clear blockages within the shunt passively.

### SUMMARY

According to a first aspect, a shunt for draining fluid from the peritoneal cavity, including a first catheter, an electronic pump fluidly coupled to the first catheter, a manual pump fluidly coupled to the electronic pump, and a second catheter fluidly coupled to the manual pump. During drainage operation, a fluid is passively pressure-driven through the shunt, and the electronic pump is arranged to prevent blockages within the shunt by flowing a bolus of the fluid through the shunt. The manual pump is arranged and configured to move the bolus of the fluid in order to inhibit blockages within the shunt when the electronic pump cannot create the bolus of fluid.

According to a second aspect, a shunt according to the previous aspect, further comprising a control unit that operates the electronic pump at discrete time intervals.

According to a third aspect, a shunt according to any of the previous aspects, wherein the electronic pump moves the bolus of fluid as a pressure wave of the fluid flowing through the shunt at a greater pressure than the fluid being pressure-driven through the shunt.

According to a fourth aspect, a shunt according to any of the previous aspects, further comprising a one-way valve fluidly coupled to the second catheter.

According to a fifth aspect, a shunt according to any of the previous aspects, wherein the first catheter, the electronic pump, the manual pump, the second catheter, and the one-way valve are serially arranged to form the shunt.

According to a sixth aspect, a shunt according to any of the previous aspects, wherein the first catheter is configured to be arranged within a peritoneal space of a subject in order to drain fluid from the peritoneal space.

According to a seventh aspect, a shunt according to any of the previous aspects, wherein the second catheter is configured to be arranged within a bladder of the subject in order to fluidly couple the peritoneal space of the subject with the bladder of the subject.

According to an eighth aspect, a shunt according to any of the previous aspects, wherein the one-way valve inhibits fluid within the bladder from flowing through the shunt.

According to a ninth aspect, wherein the manual pump is arranged and configured to move a bolus of the fluid through the shunt based on a manual activation.

According to a tenth aspect, a shunt for draining a fluid from a peritoneal cavity, including a first catheter, an electronic pump, a manual pump, a second catheter, and a one-way valve. The first catheter is configured to be arranged within the peritoneal cavity of a subject. The electronic pump is fluidly coupled to the first catheter. The manual pump is fluidly coupled to the first catheter. The second catheter is configured to be arranged within a bladder of a subject and is fluidly coupled to the electronic pump and the manual pump. A one-way valve is fluidly coupled to the second catheter and configured to inhibit a back-flow of the fluid from the bladder through the second catheter. The electronic pump is arranged and configured to move a bolus of the fluid in order to inhibit blockages within the shunt. The manual pump is arranged and configured to move a bolus of the fluid in order to inhibit blockages within the shunt when the electronic pump cannot create the bolus of fluid.

According to an eleventh aspect, a shunt according to any of the previous aspects, further comprising a control unit that operates the electronic pump at preselected time intervals.

According to a twelfth aspect, a shunt according to any of the previous aspects, wherein the electronic pump moves the bolus of fluid as a pressure wave of the fluid flowing through the shunt at a greater pressure than the fluid being passively pressure-driven through the shunt.

According to a thirteenth aspect, a shunt according to any of the previous aspects, wherein the electronic pump and the manual pump are arranged and configured to be outside of the peritoneal cavity.

According to a fourteenth aspect, a shunt according to any of the previous aspects, wherein the first catheter, the electronic pump, the manual pump, the second catheter, and the one-way valve are serially arranged to form the shunt.

According to a fifteenth aspect, a shunt according to any of the previous aspects, wherein the electronic pump and the manual pump are fluidly coupled in parallel.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature.

The following detailed description of the illustrative embodiments can be understood when read in conjunction with the following drawings, where like structure is indicated with like reference numerals and in which:
FIG. 1 schematically depicts a drainage shunt device, according to one or more embodiments shown or described herein;
FIG. 2 schematically depicts the drainage shunt device of FIG. 1 arranged within a subject, according to one or more embodiments shown or described herein;
FIG. 3A schematically depicts a drainage shunt device, according to one or more embodiments shown or described herein;
FIG. 3B schematically depicts a drainage shunt device, according to one or more embodiments shown or described herein;
FIG. 3C schematically depicts a drainage shunt device, according to one or more embodiments shown or described herein; and
FIG. 4 depicts a method of using the drainage shunt device of FIG. 1.

### DETAILED DESCRIPTION

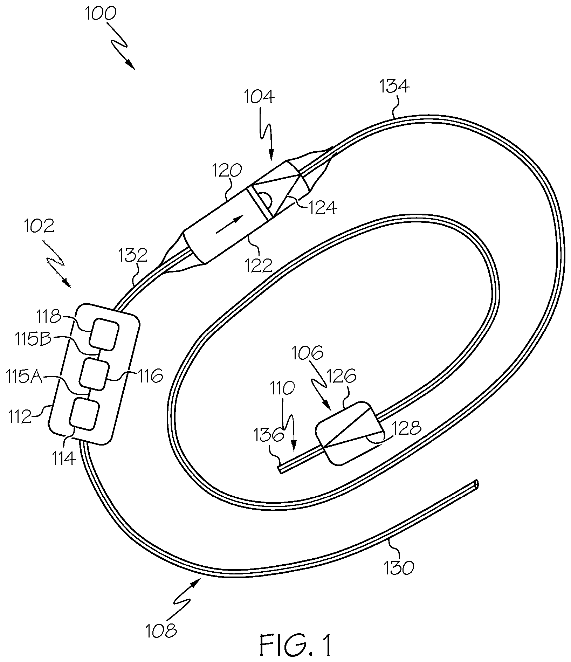

FIG. 1 generally depicts an embodiment of a shunt device for draining non-malignant ascites fluid. The shunt device generally includes an electronic pump, a manual pump, a one-way valve, a first catheter, and a second catheter. As will be described in greater detail herein, the shunt device may be used in order to drain ascites fluid from the peritoneal cavity of a subject passively to the bladder of a subject, in order to remove the ascites fluid from the subject's body. For example, the first catheter may be arranged within the peritoneal cavity, and the second catheter may be arranged within the bladder, fluidly coupling the peritoneal cavity to the bladder such that ascites fluid from the peritoneal cavity flows to the bladder due to a pressure gradient between the peritoneal cavity and the bladder and positioning of the shunt device. The term "passively" as used to describe the flow of fluid through the shunt device is defined as moving the fluid through the shunt device without the need for external pumps. The passive pumping action occurs due, at least in part, to internal forces generated by the subject's body, such as by breathing, exerting their diaphragm, a natural pressure differential between two separate cavities, and/or surface tension within the shunt device. Furthermore, the electronic pump and manual pump may be arranged to create a bolus of fluid at varying time intervals in order to clear any blockages that may have formed within the shunt device. Various embodiments of the shunt device will be described in greater detail herein.

Referring now to FIG. 1, an embodiment of a shunt device 100 is generally depicted. As illustrated, the shunt device 100 may include an electronic pump 102, a manual pump 104, a one-way valve 106, a first catheter 108, and a second catheter 110. As will be described in greater detail herein, the shunt device 100 is configured to passively drain ascites fluid from the peritoneal cavity of a subject to the bladder of the subject in order to remove the ascites fluid from the body and inhibit blockages from forming within the shunt device 100. It is noted that the present shunt device may be used to drain any type of fluid from and to any type of cavity/area within a subject's body. It is noted that the shunt device 100 may drain fluid to the bladder. However, it is contemplated that the present shunt device 100 may be used to drain fluid to other areas of the body, such as the central venous system.

Referring still to FIG. 1, the electronic pump 102 may include a housing 112, a motor 114, a control unit 116, and a battery 118. The motor 114 is communicatively coupled to the control unit 116 and the battery 118 via wires 115A, 115B. The motor 114 is fluidly coupled to the first catheter 108 such that when the motor 114 is activated by the control unit 116, a bolus of fluid formed from the fluid within the first catheter 108 is created. The control unit 116 may be a pre-programmed computer which is designed to activate the motor 114 at specific and discrete time intervals. For example, the control unit 116 may be programmed to activate the motor 114 in 12-hour increments for a duration of one minute. Additionally, the control unit 116 may be reprogrammed after surgical insertion into a subject via a wireless connection, such as Wi-Fi or Bluetooth, if the operating parameters of the electronic pump 102 need to be altered. The battery 118 of the electronic pump 102 is operatively arranged to provide power to both the motor 114 and the control unit 116. The battery 118 may be a lithium ion battery type, which may be wirelessly rechargeable through induction charging. The motor 114, control unit 116, and battery 118 are arranged within the housing 112. The housing 112 may be a rigid or flexible shell, which seals the motor 114, control unit 116, and battery 118 form the outside environment, such as inside of a subject's body. The housing 112 may be made form a non-conductive material to allow for wireless communication with the control unit 116 and induction charging of the battery 118.

The electronic pump 102 is fluidly coupled to the first catheter 108 to draw fluid to flow from the first catheter 108 through the electronic pump 102. The first catheter 108 includes a tip portion 130, which is arranged to be placed within the peritoneal cavity of a subject. In embodiments, the first catheter 108 is long enough to arrange the tip portion 130 within the peritoneal cavity of a subject, while allowing the electronic pump 102 to be arranged outside of the peritoneal cavity, but within a subject's body. The first catheter 108 may be any type of flexible tubing suitable to be inserted within a subject's body. Additionally, in embodiments, the first catheter 108 may be shaped in various forms in order to secure the first catheter 108 within a peritoneal cavity. For example, the tip portion 130 may be bent in a curve or spiral shape in order to secure the tip portion 130 within a peritoneal cavity.

Referring still to FIG. 1, the manual pump 104 may include a housing 120, a flexible membrane 122, and a valve 106. The flexible membrane 122 is used to manually pump fluid through the shunt device 100 by depressing the flexible membrane 122. By depressing the flexible membrane 122, a pressure is created within the housing 120, which causes the fluid contained within the shunt device 100 to flow towards the bladder of a subject. The fluid flows from the housing 120 through the valve 106. The valve 106 prevents back-flow of any fluid passing through the manual pump 104 from flowing back towards the peritoneal cavity. The valve 106 may be a duckbill valve, or another type of one-way valve which only allows fluid to flow in a single direction when a pressure differential is created on opposite sides of the valve 106. In embodiments, the manual pump 104 may be arranged in line with the electronic pump 102, where fluid from the peritoneal cavity would pass through the electronic pump 102 prior to the same fluid entering the manual pump 104. The manual pump 104 is fluidly coupled to the electronic pump 102 via tubing 132. Tubing 132 may be any flexible tubing which is suitable for insertion into a subject's body. In some embodiments, the manual pump 104 may be directly fluidly coupled to the electronic pump 102, without the need for tubing 132.

Referring still to FIG. 1, another one-way valve 124 is arranged to be fluidly coupled to the electronic pump 102 and the first catheter 108. The fluid flows from the housing 112 through the valve 124. The valve 124 prevents back-flow of any fluid passing through the electronic pump 102 from flowing back towards the peritoneal cavity. The valve 124 may be a duckbill valve, or another type of one-way valve which only allows fluid to flow in a single direction when a pressure differential is created on opposite sides of the valve 124.

Arranged on the opposite side of the one-way valve 106 from the manual pump 110 is the second catheter 110. The second catheter 110 includes a tip portion 136 which is inserted into the bladder of a subject. The second catheter 110 may be any type of flexible tubing suitable to be inserted within a subject's body. Additionally, in embodiments, the second catheter 110 may be shaped in various forms in order to secure the second catheter 110 within a peritoneal cavity. For example, the tip portion 136 may be bent in a curve or spiral shape in order to secure the tip portion 136 within a peritoneal cavity.

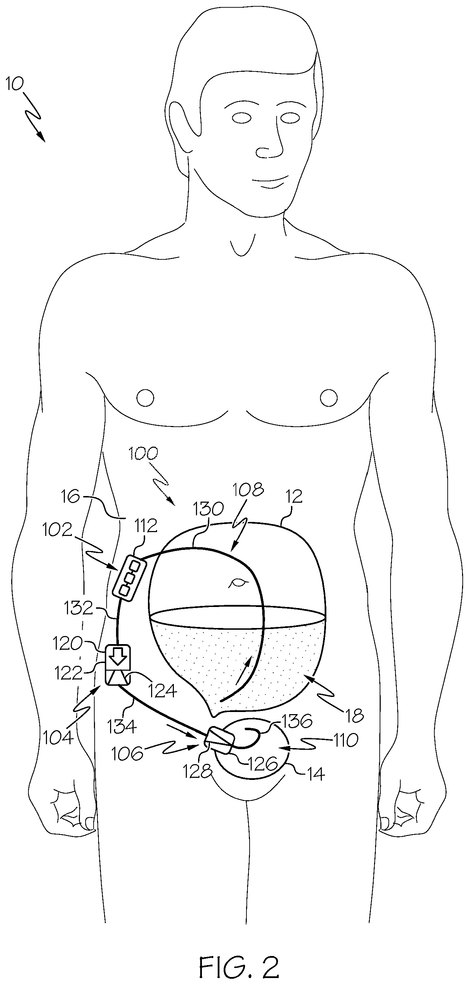

Referring now to FIG. 2, the shunt device 100 may be arranged within the body 16 of a subject 10 in order to drain ascites fluid 18 from the peritoneal cavity 12 to the bladder 14. Specifically, the first catheter 108 may be arranged within the peritoneal cavity 12 of the subject 10. The peritoneal cavity 12 is a space between the parietal peritoneum (the peritoneum that surrounds the abdominal wall) and the visceral peritoneum (the peritoneum that surrounds the internal organs) within the body 16 of the subject 10. However, in embodiments, the first catheter 108 may be arranged in any area of the body 16 which requires a fluid to be drained. The first catheter 108 is arranged within the peritoneal cavity 12 is such a way that the fluid 18 can enter the first catheter 108. For example, the tip portion 130 of the first catheter 108 may be arranged in the lower section of the peritoneal cavity 12 where the fluid 18 collects within the peritoneal cavity 12. The first catheter may also extend through an incision within the wall of the peritoneal cavity 12. The electronic pump 102 may be arranged outside of the peritoneal cavity 12, and more specifically, may be arranged just underneath the skin of the body 16 to allow for wireless charging or communication with the electronic pump 102. Additionally, the manual pump 104 may be arranged just underneath the skin of the body 16 in order to allow the subject 10 to depress the flexible membrane 122 of the manual pump 104 in order to flow a bolus of fluid through the shunt to prevent blockages.

Referring still to FIG. 2, the second catheter 110 may be arranged within the bladder 14 of the subject 10. As fluid 18 drains from the peritoneal cavity 12 to the bladder 14 through the shunt device 100, the bladder will begin to fill with the fluid 18. As the bladder 14 fills to capacity, and prior to the subject 10 emptying their bladder 14, there is the possibility that the fluid 18 stored within the bladder 14 will reach the tip portion 136 of the second catheter 110 and attempt to flow in a reverse direction through the shunt device 100. The one-way valves 106 and 124 of each pump 102 and 104 prevent the back-flow of fluid 18 from the bladder through the shunt device 100.

Due to the arrangement of the shunt device 100 within the body 16 of the subject 10, with the first catheter 108 arranged within the peritoneal cavity 12 and the second catheter 110 arranged within the bladder 14, a passive pressure-driven flow of fluid 18 can be formed between the peritoneal cavity 12 and the bladder 14 through the shunt device 100. Since the peritoneal cavity 12 and the bladder 14 are fluidly coupled, any pressure gradient between the peritoneal cavity 12 and the bladder 14 will create a pressure-driven flow of fluid 18. For example, as pressure increases within the peritoneal cavity 12, the fluid 18 stored within the peritoneal cavity 12 will be forced through the first catheter 108 as a pressure release for the peritoneal cavity 12. The pressure within the peritoneal cavity 12 may be increased by having the subject exert their diaphragm into their abdominal cavity, or by breathing normally. Since the action of breathing or exerting a diaphragm does not increase the pressure within the bladder 14, a pressure gradient is formed between the peritoneal cavity 12 and the bladder 14, with the high pressure being present in the peritoneal cavity 12, and the low pressure being present in the bladder 14. This difference in pressures passively flows the fluid 18 from the peritoneal cavity 12 to the bladder 14, without the need of additional pumping mechanisms. Due to the arrangement of the electronic pump 102 and the manual pump 104 being in fluid communication, the passive pressure driven flow may still flow through the shunt device 100 even when the pumps are not activated.

As the fluid 18 flows from the peritoneal cavity 12 to the bladder 14 through the shunt device 100, biological material may be present within the fluid and can form potential blockages within the shunt device 100 as the biological material collects on the inner surfaces of the fluid channels forming the shunt device 100. In order to prevent blockages, or to clear blockages, a bolus of fluid may be created and flowed through the components of the shunt device 100 in order to remove the biological matter to the bladder 14. A bolus of fluid is a pressure wave of the fluid 18 flowing through the shunt device 100 at a greater pressure than the fluid 18 being passively pressure-driven through the shunt device 100. The bolus of fluid may be formed by either the electronic pump 102 and/or the manual pump 104. In embodiments, the electronic pump 102 is programed to activate and create a bolus of fluid at discrete time intervals throughout the day. The purpose of having discrete time intervals to create a blockage clearing bolus is to prevent blockages from forming initially. Since the electronic pump 102 only activates for short periods of time, and only a few times per day, the battery life of the electronic pump 102 may be extended when compared to shunt devices which routinely require a pump to drain the fluid 18 from the peritoneal cavity 12 at a constant rate.

In the event that the electronic pump 102 cannot create a bolus of fluid to prevent or clear blockages within the shunt device 100, the manual pump 104 may be used to create the bolus of fluid. Situations where the electronic pump 102 may fail include a malfunction with the electrical components, the mechanical components, or a depleted battery. In these situations, without the presence of the manual pump 104, the subject 10 would be required to undergo immediate surgery to replace the electronic pump 102, otherwise excessive fluid 18 may build up within the peritoneal cavity 12 of the subject, which may cause other related health problems. The manual pump 104 may be used to create a bolus of fluid, of the same or greater pressure than that of the electronic pump 102, in order to prevent or clear blockages within the shunt device 100. If the manual pump 104 is required to be used, the subject 10 should pump the manual pump 104 at discrete time intervals throughout the day of a set number of pumps. For example, the subject should pump the manual pump 104 every twelve hours for at least twenty individual pumps. This would create a sufficient bolus of fluid 18 to clear any biological material within the shunt device 100.

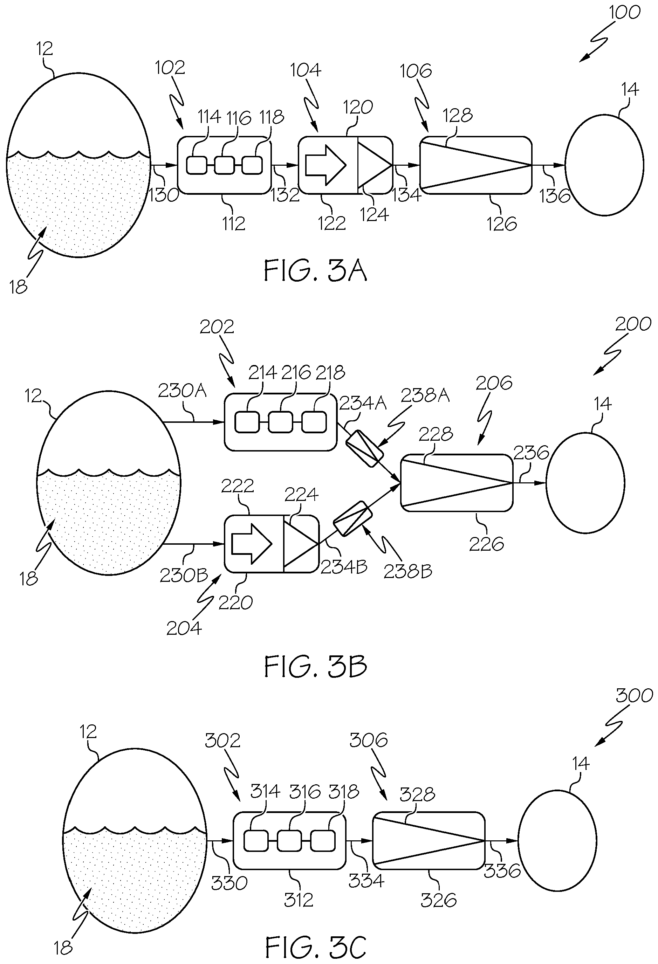

Referring now to FIGS. 3A-3C, various components forming a shunt device can be arranged to achieve different results, depending on the required parameters of the subject 10. As depicted in FIG. 3A, the electronic pump 102, manual pump 104, and one-way valve 106 are in a series arrangement, where fluid 18 from the peritoneal cavity 12 passes through each component of the shunt device 100. The fluid 18 will still pass through the electronic pump 102 and the manual pump 104 even when the pumps are not creating a bolus of fluid.

Referring now to FIG. 3B, a shunt device 200 may include an electronic pump 202 and a manual pump 204 in parallel arrangement to the peritoneal cavity 12. The electronic pump 202 is fluidly coupled to the peritoneal cavity 12 via catheter 230A. The manual pump 204 is fluidly coupled to the peritoneal cavity 12 via the catheter 230B. The electronic pump 202 is fluidly coupled to a one-way valve 224 to prevent back-flow. The manual pump 204 is fluidly coupled to a one-way valve 206 to prevent back-flow.

Referring now to FIG. 3C, a shunt device 300 may include an electronic pump 302 that includes a motor 314, a control unit 316, and a battery 318 communicatively coupled to one another in order to activate the electronic pump 302. The electronic pump 302 is fluidly coupled to the peritoneal cavity via catheter 330. The one-way valve 324 is serially arranged with the electronic pump 302 and is fluidly coupled to the electronic pump 302. The one-way valve 324 includes a housing 326 and valve members 328 to prevent back-flow of fluid 18 from the bladder 14, and is fluidly coupled to the bladder 14 via catheter 336.

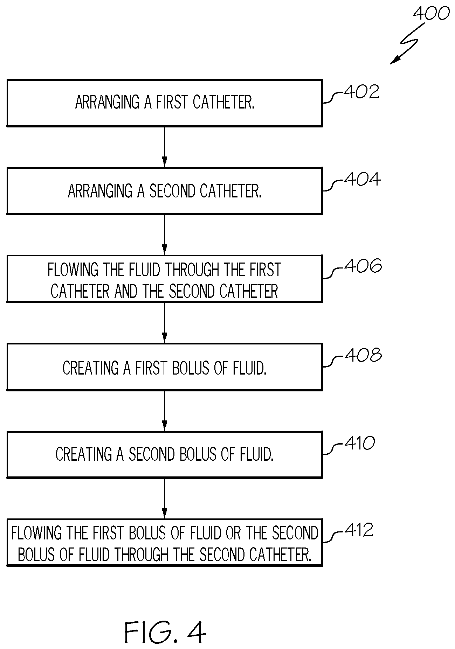

Referring now to the flow diagram of FIG. 4 in conjunction with FIGS. 1 and 2, an illustrative method 400 of draining ascites fluid from a peritoneal cavity to a bladder of a subject is schematically depicted. More specifically, a shunt device 100 is operable to passively drain fluid 18 from a peritoneal cavity 12, while also preventing blockages from forming within the shunt device 100. The depiction of FIG. 4 and the accompanying description below is not meant to limit the subject matter described herein or represent an exact description of how the shunt device 100 operates, but instead is meant to provide a simple schematic overview to illustrate the general passive drainage of fluid and blockage prevention measures of the method described herein.

Referring still to FIG. 4, in conjunction with the shunt device 100 of FIGS. 1 and 2, a flow diagram is schematically depicted of an illustrative method 400 of draining a peritoneal cavity 12 of a fluid 18. Initially, at step 402, a first catheter is arranged with a peritoneal cavity of a subject. Referring to FIG. 2, the first catheter 108 includes a tip portion 130 which may be arranged within the peritoneal cavity 12 of a subject 10. The first catheter 108 fluidly couples the remaining components of the shunt device 100 to the peritoneal cavity 12.

At step 404, a second catheter is arranged within a bladder of the subject. Referring to FIG. 2, the second catheter 110 include a tip portion 136 and is arranged within the bladder 14 of the subject 10. The first catheter 108 and the second catheter 110 are fluidly coupled to one another via the components which create the shunt device 100.

At step 406, fluid is flowed form the peritoneal cavity to the bladder. Referring to FIG. 2, the first catheter 108 and the second catheter 110 fluidly couple the peritoneal cavity 12 to the bladder 14. The fluid 18, which is present in the peritoneal cavity 12, flows through the first catheter 108 and the second catheter 110 due to a pressure differential between the peritoneal cavity 12 and the bladder 14. The pressure differential is created by the subject 10 increasing pressure within their abdominal cavity by either breathing or exerting their diaphragm into the abdominal cavity. The fluid 18 flows continuously through the first catheter 108 and the second catheter 110 in order to continuously drain the peritoneal cavity 12.

Still referring to FIG. 4, at step 408, a first bolus of fluid is created. Referring to the example being described in the preceding paragraphs, a bolus of fluid 18 may be created at discrete time intervals by the electronic pump 102 atomically in order to prevent and clear any biological material which collects within the shunt device 100. The bolus of fluid is a pressure wave of fluid 18 travelling through the second catheter 110 at a greater pressure than the passively drained fluid 18 flowing through the shunt device 100 due to the pressure differential between the peritoneal cavity 12 and the bladder 14. The greater pressure of the bolus of fluid 18 dislodges or removes any biological material present within the tubing of the shunt device 100, and removes the biological material to the bladder 14 to be expelled from the subject 10.

Still referring to FIG. 4, at step 410, a second bolus of fluid is created. Referring to the example being described in the preceding paragraphs, a second bolus of fluid 18 may be created by the manual pump 104. In embodiments, the manual pump 104 is only used in the event that the electronic pump 102 malfunctions or loses power, and is unable to produce a bolus of fluid 18. If the manual pump 104 is used to create a bolus of fluid 18, the flexible membrane 122 of the housing 120 is depressed in order to create a pressure differential between the fluid 18 within the housing 120 and the fluid within the second catheter 110. Since the pressure is greater within the housing 120, the bolus of fluid 18 will be created in the housing 120 and be biased to travel through the shunt device 100 towards the second catheter 110.

At step 412, the first bolus of fluid or the second bolus of fluid are flowed through the second catheter. Referring to the example described in the preceding paragraphs, a bolus of fluid 18 is propagated through the tubing of the shunt device 100 in order to remove any biological material which may form a blockage within the shunt device 100. Depending on the bolus creating state of the electronic pump 102, the bolus of fluid 18 may be create using the manual pump 104. If the electronic pump 102 is capable of creating a bolus of fluid 18, then the manual pump 104 would not be required to create a bolus of fluid 18.

It should now be understood that embodiments described herein are directed to a shunt device for draining non-malignant ascites fluid. The shunt device includes an electronic pump, a manual pump, a one-way valve, a first catheter, and a second catheter. The shunt device may be used in order to drain ascites fluid from the peritoneal cavity of a subject to the bladder of a subject, in order to remove the ascites fluid from the subject's body. For example, the first catheter may be arranged within the peritoneal cavity, and the second catheter may be arranged within the bladder, fluidly coupling the peritoneal cavity to the bladder. Accordingly, ascites fluid from the peritoneal cavity will flow to the bladder due to a pressure gradient between the peritoneal cavity and the bladder. Furthermore, the electronic pump and manual pump may be arranged to create a bolus of fluid at varying time intervals in order to clear any blockages that may have formed within the shunt device. The manual pump may only be required to create a bolus of fluid when the electronic pump is unable to create a bolus of fluid due to a malfunction or loss of power. The electronic pump activates automatically based on a pre-programmed time interval to allow a subject to not have to manually pump he shunt device in order to clear any biological material form the shunt device.

It will be apparent to those skilled in the art that various modifications and variations can be made to the embodiments described herein. The scope of the invention is defined by the appended claims.

## Claims

1. A shunt (100, 200, 300) for draining fluid (18) from a peritoneal cavity (12), comprising:
a first catheter (108);
an electronic pump (102, 202, 302) fluidly coupled to the first catheter;
a manual pump (104, 204) fluidly coupled to the electronic pump; and
a second catheter (110) fluidly coupled to the manual pump,
wherein, during a drainage operation, the shunt arranged and configured such that a fluid is passively pressure-driven through the shunt, and the electronic pump is arranged to move a bolus of the fluid through the shunt; and
wherein the manual pump is arranged and configured to move the bolus of the fluid in order to inhibit blockages within the shunt when the electronic pump cannot create the bolus of fluid.

2. The shunt of claim 1 further comprising a control unit (116, 316) that operates the electric pump at preselected time intervals.

3. The shunt of claim 2, wherein the electric pump moves the bolus of fluid as a pressure wave of the fluid flowing through the shunt at a greater pressure than the fluid being pressure-driven through the shunt.

4. The shunt of claim 3 further comprising a one-way valve (106, 206, 306) fluidly coupled to the second catheter.

5. The shunt of claim 4, wherein the first catheter, the electronic pump, the manual pump, the second catheter, and the one-way valve are serially arranged to form the shunt.

6. The shunt of claim 5, wherein the first catheter is configured to be arranged within a peritoneal space (12) of a subject in order to drain fluid from the peritoneal space.

7. The shunt of claim 6, wherein the second catheter is configured to be arranged within a bladder (14) of the subject in order to fluidly couple the peritoneal space of the subject with the bladder of the subject.

8. The shunt of claim 1, wherein the one-way valve inhibits fluid within the bladder from flowing through the shunt.

9. The shunt of claim 1, wherein the manual pump is arranged and configured to move a bolus of the fluid through the shunt based on a manual activation.

10. A shunt (100, 200, 300) for draining a fluid (18) from a peritoneal cavity (12), comprising:
a first catheter (108) configured to be arranged within the peritoneal cavity of a subject;
an electronic pump (102, 202, 302) fluidly coupled to the first catheter;
a manual pump (104, 204) fluidly coupled to the first catheter;
a second catheter (110) configured to be arranged within a bladder (14) of a subject, the second catheter fluidly coupled to the electronic pump and the manual pump; and,
a one-way valve (106, 206, 306) fluidly coupled to the second catheter to inhibit a back-flow of the fluid from the bladder through the second catheter,
wherein the electronic pump is arranged to create a bolus (of the fluid in order to prevent blockages within the shunt, and the manual pump is arranged and configured to move a bolus of the fluid in order to inhibit blockages within the shunt when the electronic pump cannot create the bolus of fluid.

11. The shunt of claim 10 further comprising a control unit (116, 316) that operates the electronic pump at predetermined time intervals.

12. The shunt of claim 11, wherein the electronic pump moves the bolus of fluid as a pressure wave of the fluid flowing through the shunt at a greater pressure than the fluid being passively pressure-driven through the shunt.

13. The shunt of claim 12, wherein the electronic pump and the manual pump are configured to be arranged outside of the peritoneal cavity.

14. The shunt of claim 13, wherein the first catheter, the electronic pump, the manual pump, the second catheter, and the one-way valve are serially arranged to form the shunt.

15. The shunt of claim 14, wherein the electronic pump and the manual pump are fluidly coupled in parallel.

## Patentansprüche

1. Shunt (100, 200, 300) zum Ableiten von Flüssigkeit (18) aus einer Peritonealhöhle (12), umfassend:
einen ersten Katheter (108);
eine elektronische Pumpe (102, 202, 302), die fluidisch mit dem ersten Katheter gekoppelt ist;
eine Handpumpe (104, 204), die fluidisch mit der elektronischen Pumpe gekoppelt ist; und
einen zweiten Katheter (110), der fluidisch mit der Handpumpe gekoppelt ist,
wobei der Shunt während eines Drainagevorgangs so angeordnet und konfiguriert ist, dass eine Flüssigkeit passiv unter Druck durch den Shunt getrieben wird, und die elektronische Pumpe so angeordnet ist, dass sie einen Bolus der Flüssigkeit durch den Shunt bewegt; und
wobei die Handpumpe so angeordnet und konfiguriert ist, dass sie den Bolus der Flüssigkeit bewegt, um Blockaden innerhalb des Shunts zu verhindern, wenn die elektronische Pumpe den Bolus der Flüssigkeit nicht erzeugen kann.

2. Shunt nach Anspruch 1, weiter umfassend eine Steuereinheit (116, 316), die die elektrische Pumpe in vorgewählten Zeitintervallen antreibt.

3. Shunt nach Anspruch 2, wobei die elektrische Pumpe den Bolus der Flüssigkeit als Druckwelle der durch den Shunt fließenden Flüssigkeit mit einem höheren Druck bewegt als die unter Druck durch den Shunt getriebene Flüssigkeit.

4. Shunt nach Anspruch 3, weiter umfassend ein Einwegventil (106, 206, 306), das fluidisch mit dem zweiten Katheter gekoppelt ist.

5. Shunt nach Anspruch 4, wobei der erste Katheter, die elektronische Pumpe, die Handpumpe, der zweite Katheter und das Einwegventil in Reihe angeordnet sind, um den Shunt zu bilden.

6. Shunt nach Anspruch 5, wobei der erste Katheter so konfiguriert ist, dass er innerhalb eines Peritonealraums (12) eines Patienten angeordnet werden kann, um Flüssigkeit aus dem Peritonealraum abzuleiten.

7. Shunt nach Anspruch 6, wobei der zweite Katheter so konfiguriert ist, dass er innerhalb einer Blase (14) des Patienten angeordnet werden kann, um den Peritonealraum des Patienten fluidisch mit der Blase des Patienten zu koppeln.

8. Shunt nach Anspruch 1, wobei das Einwegventil verhindert, dass Flüssigkeit in der Blase durch den Shunt fließt.

9. Shunt nach Anspruch 1, wobei die Handpumpe so angeordnet und konfiguriert ist, dass sie basierend auf einer manuellen Aktivierung einen Bolus der Flüssigkeit durch den Shunt bewegt.

10. Shunt (100, 200, 300) zum Ableiten einer Flüssigkeit (18) aus einer Peritonealhöhle (12), umfassend:
einen ersten Katheter (108), der so konfiguriert ist, dass er innerhalb der Peritonealhöhle eines Patienten angeordnet werden kann;
eine elektronische Pumpe (102, 202, 302), die fluidisch mit dem ersten Katheter gekoppelt ist;
eine Handpumpe (104, 204), die fluidisch mit dem ersten Katheter gekoppelt ist;
einen zweiten Katheter (110), der so konfiguriert ist, dass er in einer Blase (14) eines Patienten angeordnet werden kann, wobei der zweite Katheter fluidisch mit der elektronischen Pumpe und der Handpumpe gekoppelt ist; und,
ein Einwegventil (106, 206, 306), das fluidisch mit dem zweiten Katheter gekoppelt ist, um einen Rückfluss der Flüssigkeit aus der Blase durch den zweiten Katheter zu verhindern,
wobei die elektronische Pumpe so angeordnet ist, dass sie einen Bolus (der Flüssigkeit erzeugt, um Blockaden innerhalb des Shunts zu verhindern, und die Handpumpe so angeordnet und konfiguriert ist, dass sie einen Bolus der Flüssigkeit bewegt, um Blockaden innerhalb des Shunts zu verhindern, wenn die elektronische Pumpe den Bolus der Flüssigkeit nicht erzeugen kann.

11. Shunt nach Anspruch 10, weiter umfassend eine Steuereinheit (116, 316), die die elektronische Pumpe in vorbestimmten Zeitintervallen antreibt.

12. Shunt nach Anspruch 11, wobei die elektronische Pumpe den Bolus der Flüssigkeit als Druckwelle der durch den Shunt fließenden Flüssigkeit mit einem höheren Druck bewegt als die passiv unter Druck durch den Shunt getriebene Flüssigkeit.

13. Shunt nach Anspruch 12, wobei die elektronische Pumpe und die Handpumpe so konfiguriert sind, dass sie außerhalb der Peritonealhöhle angeordnet werden können.

14. Shunt nach Anspruch 13, wobei der erste Katheter, die elektronische Pumpe, die Handpumpe, der zweite Katheter und das Einwegventil in Reihe angeordnet sind, um den Shunt zu bilden.

15. Shunt nach Anspruch 14, wobei die elektronische Pumpe und die Handpumpe fluidisch parallel gekoppelt sind.

## Revendications

1. Shunt (100, 200, 300) pour drainer un fluide (18) d'une cavité péritonéale (12), comprenant :
un premier cathéter (108) ;
une pompe électronique (102, 202, 302) couplée fluidiquement au premier cathéter ;
une pompe manuelle (104, 204) couplée fluidiquement à la pompe électronique ; et
un deuxième cathéter (110) couplé fluidiquement à la pompe manuelle,
dans lequel, pendant une opération de drainage, le shunt est agencé et configuré de sorte qu'un fluide est entraîné passivement par pression à travers le shunt, et la pompe électronique est agencée pour déplacer un bolus du fluide à travers le shunt ; et
dans lequel la pompe manuelle est agencée et configurée pour déplacer le bolus du fluide afin d'inhiber des obstructions à l'intérieur du shunt lorsque la pompe électronique ne peut pas créer le bolus de fluide.

2. Shunt selon la revendication 1 comprenant en outre une unité de commande (116, 316) qui fait fonctionner la pompe électrique à des intervalles de temps présélectionnés.

3. Shunt selon la revendication 2, dans lequel la pompe électrique déplace le bolus de fluide sous la forme d'une onde de pression du fluide circulant à travers le shunt à une pression supérieure à celle du fluide étant entraîné par la pression à travers le shunt.

4. Shunt selon la revendication 3 comprenant en outre une valve unidirectionnelle (106, 206, 306) couplée fluidiquement au deuxième cathéter.

5. Shunt selon la revendication 4, dans lequel le premier cathéter, la pompe électronique, la pompe manuelle, le deuxième cathéter et la valve unidirectionnelle sont agencés en série pour former le shunt.

6. Shunt selon la revendication 5, dans lequel le premier cathéter est configuré pour être agencé à l'intérieur d'un espace péritonéal (12) d'un sujet afin de drainer du fluide de l'espace péritonéal.

7. Shunt selon la revendication 6, dans lequel le deuxième cathéter est configuré pour être agencé à l'intérieur d'une vessie (14) du sujet afin de coupler fluidiquement l'espace péritonéal du sujet avec la vessie du sujet.

8. Shunt selon la revendication 1, dans lequel la vanne unidirectionnelle empêche du fluide à l'intérieur de la vessie de s'écouler à travers le shunt.

9. Shunt selon la revendication 1, dans lequel la pompe manuelle est agencée et configurée pour déplacer un bolus du fluide à travers le shunt sur la base d'une activation manuelle.

10. Shunt (100, 200, 300) pour drainer un fluide (18) d'une cavité péritonéale (12), comprenant :
un premier cathéter (108) configuré pour être agencé à l'intérieur de la cavité péritonéale d'un sujet ;
une pompe électronique (102, 202, 302) couplée fluidiquement au premier cathéter ;
une pompe manuelle (104, 204) couplée fluidiquement au premier cathéter ;
un deuxième cathéter (110) configuré pour être agencé à l'intérieur d'une vessie (14) d'un sujet, le deuxième cathéter étant couplé fluidiquement à la pompe électronique et à la pompe manuelle ; et,
une valve unidirectionnelle (106, 206, 306) couplée fluidiquement au deuxième cathéter pour empêcher un reflux du fluide depuis la vessie à travers le deuxième cathéter,
dans lequel la pompe électronique est agencée pour créer un bolus (du fluide afin d'éviter des obstructions à l'intérieur du shunt, et la pompe manuelle est agencée et configurée pour déplacer un bolus du fluide afin d'inhiber des obstructions à l'intérieur du shunt lorsque la pompe électronique ne peut pas créer le bolus de fluide.

11. Shunt selon la revendication 10 comprenant en outre une unité de commande (116, 316) qui fait fonctionner la pompe électronique à des intervalles de temps prédéterminés.

12. Shunt selon la revendication 11, dans lequel la pompe électronique déplace le bolus de fluide sous la forme d'une onde de pression du fluide circulant à travers le shunt à une pression supérieure à celle du fluide étant entraîné passivement par la pression à travers le shunt.

13. Shunt selon la revendication 12, dans lequel la pompe électronique et la pompe manuelle sont configurées pour être agencées à l'extérieur de la cavité péritonéale.

14. Shunt selon la revendication 13, dans lequel le premier cathéter, la pompe électronique, la pompe manuelle, le deuxième cathéter et la valve unidirectionnelle sont agencés en série pour former le shunt.

15. Shunt selon la revendication 14, dans lequel la pompe électronique et la pompe manuelle sont couplées fluidiquement en parallèle.
